(19) Europäisches Patentamt
European Patent Office
Office européen des brevets

(11) Veröffentlichungsnummer: **0 000 554**
**B1**

(12) **EUROPÄISCHE PATENTSCHRIFT**

(45) Veröffentlichungstag der Patentschrift: **10.02.82**

(21) Anmeldenummer: **78100456.9**

(22) Anmeldetag: **20.07.78**

(51) Int. Cl.³: **C 07 D 303/08, C 07 D 301/14**

(54) **Verfahren zur Herstellung von halogenalkylsubstituierten Oxiranen.**

(30) Priorität: **28.07.77 DE 2734085**

(43) Veröffentlichungstag der Anmeldung:
**07.02.79 Patentblatt 79/3**

(45) Bekanntmachung des Hinweises auf die Patenterteilung:
**10.02.82 Patentblatt 82/6**

(84) Benannte Vertragsstaaten:
**BE CH DE FR GB NL**

(56) Entgegenhaltungen:
**FR - A - 2 300 085**
**FR - A - 2 369 273**
**FR - A - 2 369 274**

(73) Patentinhaber: **BAYER AG**
**Zentralbereich Patente, Marken und Lizenzen**
**D-5090 Leverkusen 1, Bayerwerk (DE)**

(73) Patentinhaber: **Degussa Aktiengesellschaft**
**Weissfrauenstrasse 9**
**D-6000 Frankfurt am Main (DE)**

(72) Erfinder: **Rauleder, Gebhard, Dr.**
**Cheruskerstrasse 31**
**D-4000 Düsseldorf (DE)**
Erfinder: **Seifert, Hermann, Dr.**
**Ruwergasse 4**
**D-5000 Köln 80 (DE)**
Erfinder: **Kastenhuber, Hubert, Dr.**
**Stettinerstrasse 6**
**D-5040 Brühl (DE)**
Erfinder: **Prescher, Günter, Dr.**
**Grünaustrasse 11**
**D-6450 Hanau (DE)**

**EP 0 000 554 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung von halogenalkylsubstituierten Oxiranen

Die vorliegende Erfindung betrift ein verbessertes Verfahren zur Herstellung von halogenalkylsubstituierten Oxiranen aus halogenalkylsubstituierten Olefinen und Percarbonsäuren.

Halogenalkylsubstituierte Oxirane finden Verwendung auf dem Gebiet der Lacke und Kunststoffe und also organische Zwischenprodukte.

Es ist bekannt, chloralkylsubstituierte Oxirane aus den entsprechenden Olefinen nach dem Chlorhydrinverfahren herzustellen. Dieses Verfahren hat den Nachteil, daß unerwünschte chlorierte Nebenprodukte und umweltbelastende Salzabfälle gebildet werden (Ullmanns Encycklopädie d. techn. Chemie, Bd. 10, S. 565, linke Spalte, Zeile 1 ff., insbesondere 3. 13—15; DAS 1 543 174, Spalte 2, Zeile 15 ff., insbesondere 3. 32—35).

Häufig ist es auch schwierig, nach der Chlorhydrinmethode definiert ein einziges Reaktionsprodukt herzustellen. So führt die Umsetzung von 4-Chlorbuten-(2) mit unterchloriger Säure zu einem Gemisch von zwei Produkten, die durch nachstehende Formeln gekennzeichnet seien:

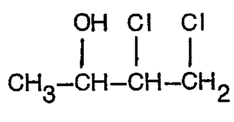 und 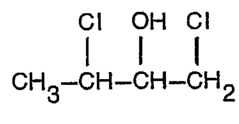

Demgemäß liefert die nachfolgende Dehydrohalogenierung dieses Gemisches mit einer Base ein Gemisch von zwei isomeren Oxiranen, wie nachstehende Formeln veranschaulichen (DAS 1 056 596, Spalte 1, Zeile 53 bis Spalte 2, 3—43):

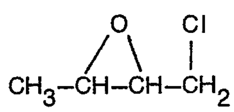 und 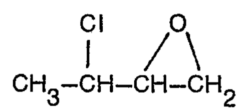

Weiterhin ist es bekannt, Olefine mit Hilfe einer Percarbonsäure in die entsprechenden Oxirane umzuwandeln. (N. Prileschajew, Ber, dtsch. Chem. Ges. *42,* 4811 (1909)).

Bei dieser Reaktion handelt es sich um einen elektrophilen Angriff des Oxidationsmittels auf das Olefin. (K. D. Bingham, G. D. Meakins, G. H. Whitham, Chem. Commun. (1966, S 445 und 446).) Aus diesem Grunde nimmt die Reaktivität des Olefins mit fallender Nucleophilie der Doppelbindung ab. Deshalb erschweren elektronegative Substituenten in $\alpha$-Stellung zur C=C—Doppelbindung die Epoxidation, (S. N. Lewis in R. L. Augustin, "Oxidation", Vol. I, Seite 227, insbesondere S. 227, 3. 9—13, Marcel Dekker, New York (1969).) Halogenalkylsubstituierte Olefine lassen sich daher mit Percarbonsäuren nicht ohne weiteres epoxidieren. Infolge der geringen Reaktionsfähigkeit ihrer Doppelbindung sind hohe Temperaturen und lange Reaktionszeiten erforderlich, was zur Bildung von unerwünschten Nebenprodukten wie Dihydroxyund Hydroxyacyloxy-Derivaten der Ausgangsprodukte Anlaß gibt. (S. N. Lewis in R. L. Augustin, "Oxidation", Vol. I, Seite 233, insbesondere 3. 6—11, Marcel Dekker, New York 1969).

So ist insbesondere im Hinblick auf Art und Durchführung der Reaktion zwischen einem halogenalkylsubstituierten Olefin und einer Percarbonsäure die Struktur und Herstellungsweise der verwendeten Percarbonsäure von großer Bedeutung.

Bekanntlich lassen sich niedere aliphatische Percarbonsäuren in einer Gleichgewichtsreaktion aus Carbonsäure und Wasserstoffperoxid gemäß Gleichung (1) herstellen. (D. Swern, in "Organic Peroxides", Vol. 1, S. 61, Wiley Intersciense 1971).

$$RCOOH + H_2O_2 \rightleftharpoons RCOOOH + H_2O \qquad (1)$$

Mit Ausnahme von stärkeren Carbonsäuren wie Ameisensäure und Trifluoressigsäure benötigt man zur schnellen Einstellung des Gleichgewichtes starke Säuren, wie Schwefelsäure, p-Toluolsulfonsäure und andere, als Katalysator (S. N. Lewis in R. L. Augustin "Oxidation", Vol. I, S. 216 ("C. Peracids"), Marcel Dekker, New York 1969). Die Umsetzung von Olefinen mit z.B. nach dieser Methode hergestellter Peressigsäure führte jedoch nicht zu Oxiranen, sondern zu $\alpha$-Glykolen und Hydroxyacetaten (J. Böseken, W. C. Smit und Gaster, Proc. Acad. Sci. Amsterdam, *32* 377—383 (1929).) Die im Reaktionsgemisch vorhandene Mineralsäure katalysiert die Aufspaltung des primär gebildeten Oxirans (D. Swern "Organic Peroxides", Wiley Intersciense 1971, Vol. 2, S. 436), was besonders bei reaktionsträgen Olefinen, wie halogenalkylsubstituierten Olefinen, bei deren Umsetzung hohe Temperaturen und lange Reaktionszeiten nötig sind, zu Oxiranverlusten führen kann.

Perameisensäure läßt sich ohne zusätzlichen Katalysator aus Wasserstoffperoxid und Ameisensäure herstellen (S. N. Lewis in R. L. Augustin, "Oxidation", Vol. I, S. 217, erster Absatz, Marcel Dekker, New York 1969). Die Umsetzung von $\alpha$-chloralkylsubstituierten Olefinen mit dieser mineralsäurefreien Percarbonsäure lieferte jedoch das entsprechende Epoxid auch nur zu niedrigen Ausbeuten. So wurde

zur Epoxidation von 3,4-Dichlorbuten-(1) eine aus 90%-iger Ameisensäure und 85%-igem Wasserstoffperoxid hergestellte Perameisensäure verwendet. Nach fünf Stunden Reaktionsdauer bei 60°C wurde 2-(1,2-Dichloräthyl-)oxiran in 30%-iger Ausbeute erhalten (E. G. E. Hawkins, J. Chem. Soc., 1959 S. 248 bis 256, insbesondere S. 250, Z. 19).

In neuerer Zeit ist ein verfahren zur Herstellung von aliphatischen Chlorepoxiden durch Umsetzung eines Allylchlorkohlenwasserstoffs, welcher ein Chloratom in Nachbarstellung zur Doppelbindung besitzt, mit einer organischen Perverbindung, die frei von anorganischen Verunreinigungen ist, bekannt geworden (DAS 1 056 596). Die dabei verwendeten Perverbindungen sind "reine Peressigsäure, Perpropionsäure oder Acetaldehydmonoperacetat im Gemisch mit Acetaldehyd und/oder Aceton". (DAS 1 056 596, Spalt 10, Z. 32—35.) Die Epoxidierung gemäß dem Verfahren der DAS 1 056 596 von in Allylstellung chlorsubstituierten Olefinen mit Acetaldehydmonoperacetat liefert die entsprechenden Oxirane in Ausbeuten bezogen auf die Perverbindung je nach Olefin zwischen 17% und 56%. (DAS 1 056 596, Spalte 5 bis 7, Zeile 35 ff., Beispiel 1, 3, 4 und 6).

Die bei diesem Verfahren zur Epoxidation verwendete Peressigsäure und Perpropionsäure wird gelöst in einem inerten organischen Lösungsmittel eingesetzt. Wie an anderer Stelle beschrieben, können bei diesem Verfahren als typische inerte Lösungsmittel unter anderem Aceton, Äthylacetat, Butylacetat und Dibutyläther verwendet werden (US—P. 3 150 154, Spalte 3, Zeile 1—3).

Mit den gemäß dem Verfahren der DAS 1 056 596 hergestellten Persäuren lassen sich Allylchlorkohlenwasserstoffe epoxidieren; die Ausbeuten an Oxiranen sind jedoch niedrig; der Persäure-Umsatz ist unvollständig. Er beträgt in den angegebenen Beispielen nur etwa 90% und die Reinheit der isolierten Oxirane ist für Technische Verwendung ungenügend. So wird in der DAS 1 056 596 im Beispiel 5, Spalte 7, Zeile 5 ff. die Epoxidation von 3-Chlor-1-buten mit einer Lösung von Peressigsäure in Aceton beschrieben. Der persäure-Umsatz beträgt nach zehnstündiger Reaktionsdauer 91%. Das Oxiran wird mit einer Reinheit von 90,5% in 68%-iger Ausbeute isoliert.

In der Brit. PS 784 620 wird im Beispiel VII, Seite 7, Zeile 5 ff., die Herstellung von 3,4-Dichloro-1,2-epoxybutan durch Umsetzung von 3,4-Dichlor-1-buten mit Peressigsäure in Aceton beschrieben. Danach beträgt der Persäure-Umsatz 89% und die Epoxid-Ausbeute 75%. Die Reinheit des Epoxids wird mit 93,3% angegeben. In der Brit. PS 784 620 wird im Beispiel IX, Seite 7, Zeile 85 ff. auch über die Epoxidation eines Olefins mit Perpropionsäure berichtet. Danach wurde nach der Umsetzung von Crotylchlorid mit einer Lösung von Perpropionsäure in Äthylpropionat das 1-Chloro-2,3-epoxybutan in 56%-iger Ausbeute erhalten. Der Persäure-Umsatz betrug 90%.

In der französischen Patentanmeldung mit der Veröffentlichungs-Nr. 2 300 085 ist als einziges, nach dem dort beschriebenen Verfahren epoxidierbares Halogen-haltiges Olefin, Allylchlorid beschrieben. Dem Fachmann ist geläufig, daß bereits ein elektronegativer Substituent, beispielsweise ein Chlor- oder Bromatom, die Epoxidation des Olefins erschwert. Es konnte deshalb nicht erwartet werden, daß bei der Epoxidation von mindestens zwei Chlor- oder Bromatome enthaltenden Olefinen mit Persäuren Epoxide in Ausbeuten von über 90% (bezogen auf eingesetzte Persäure) erhalten werden können, wie dies entsprechend der vorliegenden Erfindung gelingt.

Demgegenüber wurde nun gefunden, daß man halogenalkylsubstituierte Oxirane aus halogenalkylsubstituierten Olefinen und Percarbonsäuren herstellen kann, wenn man ein chloralkyl- oder bromalkylsubstituiertes Monoolefin der allgemeinen Formel

$$R_1-\overset{\displaystyle R_2}{\overset{|}{C}}=\overset{\displaystyle R_3}{\overset{|}{C}}-R_4, \qquad (I)$$

worin

$R_1$ und $R_4$ unabhängig voneinander Wasserstoff, $C_1$- bis $C_5$-Alkyl, $C_5$- bis $C_7$-Cycloalkyl, Monochlor-$C_1$- bis $C_5$-Alkyl, Monobrom-$C_1$-bis $C_5$-Alkyl, Dichlor-$C_1$- bis $C_5$-Alkyl, Dibrom-$C_1$- bis $C_5$-Alkyl, Monochlor-$C_5$-bis $C_7$-Cycloalkyl, Monobrom-$C_5$- bis $C_7$-Cycloalkyl, Dichlor-$C_5$- bis $C_7$-Cycloalkyl, Dibrom-$C_5$- bis $C_7$-Cycloalkyl bedeuten,

$R_2$ und $R_3$ unabhängig voneinander für wasserstoff, $C_1$- bis $C_5$-Alkyl, Monochlor-$C_1$- bis $C_5$-Alkyl, Monobrom-$C_1$ bis $C_5$-Alkyl, Dichlor-$C_1$- bis $C_5$-Alkyl und Dibrom-$C_1$- bis $C_5$-Alkyl stehen, wobei die Reste $R_2$ und $R_3$ zusammen mit den Kohlenstoffatomen der C=C -Doppelbindung einen Ring mit bis zu 12 Kohlenstoffatomen bilden können,

und wobei mindestens einer der Reste $R_1$ bis $R_4$ ein Dichlor- oder Dibromrest der genannten Art ist oder wobei mindestens zwei der Reste $R_1$ bis $R_4$ ein Chlor oder Brom enthaltender Rest der genannten Art sind,

und wobei das Chloralkyl- oder Bromalkylsubstituierte Monoolefin mindestens 4 Kohlenstoffatome enthält,

mit einer Lösung einer 3 bis 4 Kohlenstoffatome enthaltenden Percarbonsäure in einem Chlorierten, 1 bis 8 Kohlenstoffatome enthaltenden Kohlenwasserstoff bei einem Molverhältnis von Monoolefin zu Percarbonsäure wie 1,1:1 bis 10:1 und bei einer Temperatur von 30°C bis 100°C umsetzt, wobei die Percarbonsäure bis zu 5 Gew.-% Wasser und bis zu 2 Gew.-% Wasserstoffperoxid enthält und die zur Umsetzung gelangende Percarbonsäurelösung einen Mineralsäuregehalt unterhalb von 50 ppm besitzt.

Bevorzugt wird ein chloralkyl- oder bromalkylsubstituiertes Monoolefin mit mindestens 4 Kohlenstoffatomen eingesetzt.

Im Rahmen der Verbindungen der Formel (I) kommen beispielsweise Verbindungen der folgenden Formeln besonders in Betracht:

$$R_5\text{—}CH{=}CH\text{—}R_6, \qquad (II)$$

worin

$R_5$ und $R_6$ unabhängig voneinander $C_1$- bis $C_5$-Alkyl, Monochlor-$C_1$- bis $C_5$-Alkyl, Monobrom-$C_1$- bis $C_5$-Alkyl, Dichlor-$C_1$- bis $C_5$-Alkyl oder Dibrom-$C_1$- bis $C_5$-Alkyl bedeuten,
wobei die Reste $R_5$ und $R_6$ zusammen mit der Gruppe CH=CH zu einem Ring verknüpft sein können, und wobei mindestens einer der Reste $R_5$ und $R_6$ Dichlor-$C_1$- bis $C_5$-Alkyl oder Dibrom-$C_1$- bis $C_5$-Alkyl bedeutet oder beide Reste $R_5$ und $R_6$ Monochlor-$C_1$- bis $C_5$-Alkyl oder Monobrom-$C_1$- bis $C_5$-Alkyl bedeuten;

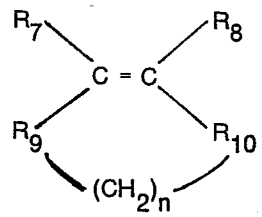

(III)

worin

$R_7$ und $R_8$ unabhängig voneinander wasserstoff, $C_1$- bis $C_5$-Alkyl, Monochlor-$C_1$- bis $C_5$-Alkyl, Monobrom-$C_1$- bis $C_5$-Alkyl, Dichlor-$C_1$- bis $C_5$- Alkyl oder Dibrom-$C_1$- bis $C_5$-Alkyl bedeuten,
$R_9$ und $R_{10}$ unabhängig voneinander einen Methylen-Chlormethylen-, Brommethylen-, 1,2-Dichloräthylen- oder 1,2-Dibrommethylen-Rest darstellen,
für eine ganze Zahl von 1 bis 6 steht, und wobei mindestens einer der Reste $R_7$ bis $R_{10}$ ein Dichlor- oder Dibromrest der genannten Art ist, oder wobei mindestens zwei der Reste $R_7$ bis $R_{10}$ ein Chlor oder Brom enthaltender Rest der genannten Art sind.

Im einzelnen seien als halogenalkylsubstituierte Monoolefine genannt:

3-Chlor-2-chlormethyl-propen, 1,4-Dichlor-2-buten, 3,4-Dichlor-1-buten, 1,4-Dichlor-2-penten, 3,4-Dichlor-1-penten, 1,2-Dichlor-3-penten, 1,4-Dichlor-2-cyclopenten, 1,4-Dichlor-2-hexen, 3,4-Dichlor-1-hexen, 2,5-Dichlor-3-hexen, 1,4-Dichlor-2-cyclohexen, 3,4-Dichlor-1-hepten, 1,4-Dichlor-2-hepten, 2,5-Dichlor-3-hepten, 1,4-Dichlor-2-cyclohepten, 1,4-Dichlor-2-octen, 2,5-Dichlor-3-octen, 3,6-Dichlor-4-octen, 1,4-Dichlor-2-cycloocten, 1,4-Dichlor-2-nonen, 2,5-Dichlor-3-nonen, 3,6-Dichlor-4-nonen, 1,4-Dichlor-2-decen, 2,5-Dichlor-3-decen, 3,6-Dichlor-4-decen, 4,7-Dichlor-5-decen, 1,4-Dichlor-2-undecen, 2,5-Dichlor-3-undecen, 4,7-Dichlor-5-undecen, 5,8-Dichlor-6-undecen, 1,4-Dichlor-2-dodecen, 2,5-Dichlor-3-dodecen, 4,7-Dichlor-5-dodecen, 5,8 Dichlor-6-dodecen, 5,7-Dichlor-6-dodecen, 3-Brom-2-brommethyl-propen, 1,4-Dibrom-2-buten, 3,4-Dibrom-1-buten, 1,4-Dibrom-2-penten, 3,4-Dibrom-1-penten, 1,2-Dibrom-3-penten, 1,4-Dibrom-2-cyclopenten, 1,4-Dibrom-2-hexen, 3,4-Dibrom-1-hexen, 2,5-Dibrom-3-hexen, 1,4-Dibrom-2-cyclohexen, 3,4-Dibrom-1-hepten, 1,4-Dibrom-2-hepten, 2,5-Dibrom-3-hepten, 1,4-Dibrom-2-cyclohepten, 1,4-Dibrom-2-octen, 2,5-Dibrom-3-octen, 3,6-Dibrom-4-octen, 1,4-Dibrom-2-cycloocten, 1,4-Dibrom-2-nonen, 2,5-Dibrom-3-nonen, 3,6-Dibrom-4-nonen, 1,4-Dibrom-2-decen, 2,5-Dibrom-3-decen, 3,6-Dibrom-4-decen, 4,7-Dibrom-5-decen, 1,4-Dibrom-2-undecen, 2,5-Dibrom-3-undecen, 4,7-Dibrom-5-undecen, 5,8-Dibrom-6-undecen, 1,4-Dibrom-2-dodecen, 2,5-Dibrom-3-dodecen, 4,7-Dibrom-5-dodecen, 5,8-Dibrom-6-dodecen, 5,7-Dibrom-6-dodecen.

Besonders geeignet zur Umsetzung mit Percarbonsäuren gemäß dem erfindungsgemäßen Verfahren sind chloralkyl- oder bromalkylsubstituierte Monoolefine der Formel

$$R_{11}\text{—}CH{=}CH\text{—}R_{12}, \qquad (IV)$$

worin

$R_{11}$ und $R_{12}$ unabhängig voneinander Wasserstoff, $C_1$- bis $C_5$-Alkyl, Monochlor-$C_1$- bis $C_5$-Alkyl, Monobrom-$C_1$- bis $C_5$-Alkyl, Dichlor-$C_1$- bis $C_5$-Alkyl oder Dibrom-$C_1$- bis $C_5$-Alkyl bedeuten,
wobei mindestens einer der Reste $R_{11}$ und $R_{12}$ für Dichlor-$C_1$- bis $C_5$-Alkyl oder Dibrom-$C_1$- bis $C_5$-Alkyl steht oder beide Reste $R_{11}$ und $R_{12}$ für Monochlor-$C_1$- bis $C_5$-Alkyl oder Monobrom-$C_1$- bis $C_5$- bis $C_5$-Alkyl stehen.

Im einzelnen seien beispielsweise genannt:

3-Chlor-2-chlormethyl-propen, 1,4-Dichlor-2-buten, 3,4-Dichlor-1-buten, 1,4-Dichlor-2-penten, 3,4-Dichlor-1-penten, 1,2-Dichlor-3-penten, 1,4-Dichlor-2-hexen, 3,4-Dichlor-1-hexen, 2,5-Dichlor-3-hexen, 1,4-Dichlor-2-cyclohexen, 3-Brom-2-brommethyl-propen, 1,4-Dibrom-2-buten, 3,4-Dibrom-1-buten, 1,4-Dibrom-2-penten, 3,4-Dibrom-1-penten, 1,2-Dibrom-3-penten, 1,4-Dibrom-2-hexen, 3,4-Dibrom-1-hexen, 2,5-Dibrom-3-hexen, 1,4-Dibrom-2-cyclohexen.

Ganz besonders sind zur Umsetzung mit Percarbonsäuren nach dem erfindungsgemäßen Verfahren 1,4-Dichlor-2-buten, 1,4-Dibrom-2-buten und 3,4-Dichlor-1-buten geeignet.

Als Lösungsmittel können die verschiedensten chlorierten Kohlenwasserstoffe verwendet werden, wie Methylenchlorid, Chloroform, Tetrachlorkohlenstoff, 1-Chloräthan, 1,2-Dichloräthan, 1,1-Dichloräthan, 1,12,2-Tetrachloräthan, 1-Chlorpropan, 2-Chlorpropan, 1,2-Dichlorpropan, 1,3-Dichlorpropan, 2,3-Dichlorpropan, 1,2,3-Trichlorpropan, 1,1,2,3-Tetrachlorpropan, Butychlorid, 1,2-Dichlorbutan, 1,4-Dichlorbutan, 2,3-Dichlorbutan, 1,3-Dichlorbutan, 1,2,3,4-Tetrachlorbutan, tert. Butylchlorid, Amylchlorid, 1,2-dichlorpentan, 1,5-Dichlorpentan, 1,2,3,4-Tetrachlorpentan, Cyclopentylchlorid, 1,2-Dichlorcyclopentylchlorid, Hexylchlorid, 1,2-Dichlorhexan, 1,6-Dichlorhexan, 1,2,3,4-Tetrachlorhexan, 1,2,5,6-Tetrachlorhexan, Cyclohexylchlorid, 1,2-Dichlorhexan, Chlorbenzol, Heptylchlorid, 1,2-Dichlorheptan, 1,2,3,4-Tetrachlorheptan, Cycloheptylchlorid, 1,2-Dichlorheptan, Octylchlorid, 1,2-Dichloroctan, 1,2,3,4-Tetrachloroctan, Cyclooctylchlorid, und 1,2-Dichloroctan.

Bevorzugte Lösungsmittel sind Methylenchlorid, Chloroform, Tetrachlorkohlenstoff und 1,2-Dichlorpropan. Besonders bevorzugtes Lösungsmittel ist 1,2-Dichlorpropan. Verwendet werden können auch Lösungsmittelgemische chlorierter Kohlenwasserstoffe.

Erfindungsgemäß verwendbare Persäuren sind Perpropionsäure, Perbuttersäure und Perisobuttersäure. Bevorzugt verwendet werden Perpropionsäure und Perisobuttersäure. Besonders bevorzugt ist Perpropionsäure. Die Herstellung der mineralsäurefreien Persäuren in einem der genannten organischen Lösungsmittel kann z.B. nach dem in der DOS 2 262 970 beschriebenen Verfahren erfolgen.

Das erfindungsgemäße Verfahren wird in einem Temperaturbereich von 30—100°C durchgeführt. Bevorzugt arbeitet man bei 60—80°C, besonders bevorzugt bei 65—75°C. In Sonderfällen können auch die angegebenen Temperaturen unter- oder überschritten werden.

Neben der Arbeitsweise unter isothermen Bedingungen, d. h. Einhaltung einer einheitlichen Temperatur im gesamten Reaktionsgemisch, kann man die Umsetzung auch unter Ausbildung eines sogenannten Temperaturgradienten durchführen, der im allgemeinen mit fortschreitender Reaktion zunimmt. Man kann aber auch die Reaktion so führen, daß sich mit dem Fortschreiten der Reaktion ein Gradient fallender Temperatur ausbildet.

Das Molverhältnis von Olefin zu Persäure beträgt erfindungsgemäß 1,1 : 1 bis 10 : 1. Bevorzugt wird ein Molverhältnis von 1,25 : 1 bis 5 : 1 angewendet. Ganz besonders vorteilhaft ist es, ein Molverhältnis von 1,5 bis 3,0 Mol Olefin je Mole Persäure anzuwenden.

Das erfindungsgemäße Verfahren kann bei den verschiedensten Drücken durchgeführt werden. Im allgemeinen arbeitet man bei Normaldruck; das Verfahren kann jedoch auch bei Unter- oder Überdruck durchgeführt werden.

Der Wassergehalt der zur Epoxidation verwendeten Percarbonsäure beträgt bis 5 Gew.-%. Geeignet ist beispielsweise eine Percarbonsäure mit einem Wassergehalt von bis zu 2 Gew.-%. Vorzugsweise verwendet man eine Percarbonsäurelösung, die weniger als 1 Gew.-% Wasser enthält. Besonders bevorzugt ist ein Wassergehalt von weniger als 0,1 Gew.-%.

Der Wasserstoffperoxidgehalt der angewendeten Percarbonsäure beträgt bis zu 2 Gew.-%. Vorteilhaft arbeitet man bei einem Gehalt von weniger als 1 Gew.-%. Besonders vorteilhaft ist es, die Umsetzung mit einer Percarbonsäurelösung durchzuführen, die einen Wasserstoffperoxidgehalt unter 0,3 % aufweist.

Der Mineralsäuregehalt der zur Umsetzung gelangenden Percarbonsäurelösung liegt unterhalb 50 ppm. Besonders vorteilhaft ist ein Mineralsäuregehalt von weniger als 10 ppm.

Die Durchführung der Reaktion kann diskontinuierlich oder kontinuierlich in den für Umsetzungen dieser Art üblichen Vorrichtungen wie Rührwerkskessel Siedereaktoren, Röhrenreaktoren, Schlaufenreaktoren oder Schleifenreaktoren erfolgen.

Als Werkstoffe für die Durchführung der Verfahren können Glas, Edelstähle oder emailliertes Material verwendet werden.

Schwermetallionen im Reaktionsgemisch katalysieren die Zersetzung der Percarbonsäure. Man setzt deshalb der Percarbonsäurelösung im allgemeinen Substanzen zu, die die Schwermetallionen durch Komplexbildung inaktivieren. Bekannte Substanzen solcher Art sind Gluconsäure, Äthylendiamintetraessigsäure, Natriumsilicat, Natriumpyrophosphat, Natriumhexametaphosphat, Dinatriumdimethylpyrophosphat, oder $Na_2$ (2-Äthyl-hexyl)$_5$($P_3O_{10}$)$_2$ (DAS 1 056 596, Spalte 4, Zeile 60 ff.).

Das halogenalkylsubstituierte Olefin kann auf verschiedene Art in die für die Umsetzung verwendete Vorrichtung eingebracht werden. Man kann es gemeinsam mit der Percarbonsäurelösung in den Reaktor geben oder man führt die beiden Komponenten getrennt voneinander dem Reaktor zu. Weiterhin ist es möglich, das Olefin und die Percarbonsäurelösung an verschiedenen Stellen in die Reaktoreinheit zu leiten. Bei Verwendung mehrerer in Kaskade geschalteter Reaktoren kann es zweckmäßig sein, das gesamte Olefin in den ersten Reaktor einzubringen. Man kann aber das Olefin auch auf die verschiedenen reaktoren aufteilen.

Die Reaktionswärme wird durch innen- oder außenliegende Kühler abgeführt. Zur Ableitung der Reaktionswärme kann die Umsetzung auch unter Rückfluß (Siedereaktoren) durchgeführt werden.

Die Reaktion wird zweckmäßigerweise unter möglichst vollständiger Umsetzung der Percarbonsäure vorgenommen. Im allgemeinen setzt man mehr als 95 Mol-% der Percarbonsäure um. Zweckmäßig ist es, mehr als 98 Mol-% Persäure umzusetzen.

Bei der erfindungsgemäßen Durchführung der Reaktion zwischen dem halogenalkylsubstituierten Olefin und der Persäure gelingt es, Oxiranausbeuten von 90 % der Theorie und darüber, bezogen auf eingesetzte Percarbonsäure, zu erzielen.

Die Aufarbeitung des reaktionsgemisches erfolgt in an sich bekannter Weise, z. B. durch Destillation. Besonders vorteilhaft ist es, das Reaktionsgemisch vor der destillativen Aufarbeitung zur Abtrennung der bei der Umsetzung gebildeten, der Percarbonsäure entsprechenden Carbonsäure mit Wasser zu extrahieren. Die Durchführung der Extraktion kann in denn üblichen Extraktoren, wie Mischer-Scheider, Siebbodenextraktoren, pulsierende Siebbodenkolonnen, Drehscheibenextraktoren oder Extraktionszentifrugen erfolgen.

Bei einer vevorzugten Durchführung des Verfahrens wird eine etwa 20 Gew.-%ige Perpropionsäurelösung in 1,2-Dichlorpropan unter Rühren zu der dreifach-molaren Menge halogenalkyl-substituiertem Olefin, das auf 70°C thermostatisiert ist, gegeben. Die Perpropionsäurelösung enthält weniger als 10 ppm Mineralsäure; sie hat einen Wassergehalt, der unterhalb von 0,1 % liegt und weist einen Wasserstoffperoxidgehalt von weniger als 0,3 % auf. Zur Komplexierung von Schwermetallionen wurde der Perpropionsäure vor der Umsetzung etwa 0,05 Gew.-% $Na_5$ (2-Äthylhexyl)$_5$($P_3O_{10}$)$_2$ zugesetzt. Der Fortgang und das Ende der Reaktion werden kontrolliert, indem man der Reaktionslösung in zeitlichen Abständen Proben entnimmt und titrimetrisch den noch vorhandenen Gehalt an Percarbonsäure bestimmt. Nach beendigung der Reaktion wird das Reaktionsgemisch abgekühlt und zur Entfernung der Propionsäure dreimal mit derselben Gewichtsmenge Wasser gewaschen. Anschließend wird das propionsäurefreie Reaktionsgemisch fraktioniert.

Die folgenden Beispiele erläutern die Erfindung. Sämtliche Prozentangaben stellen, soweit nichts anderes gesagt wird, Gewichtsprozent dar.

Beispiel 1.

Herstellung von 2-(1,2-Dichloräthyl-)oxiran aus 3,4-Dichlor-1-buten und Perpropionsäure.

In 55,87 g (0,447 Mol) 3,4-Dichlor-1-buten tropfte man bei 70°C unter Rühren 62,96 g (0,147 Mol) Perpropionsäure als 21 %ige Lösung in 1,2-Dichlorpropan. Nach Zutropfen wurde noch weitere 6 Stunden bei dieser Temperatur gerührt, dann zeigte die titrimetrische Analyse einen Perpropionsäure-Umsatz von 95 %. Die Reaktionslösung wurde auf Raumtemperatur abgekühlt und gaschromatographisch analysiert. Wie die Analyse zeigte, wurde 2-(1,2-Dichloräthyl-)-oxiran mit einer Selektivität, bezogen auf eingesetzte Perpropionsäure, von 92,6 % gebildet.

Das Reaktionsgemisch wurde zur Entfernung der Propionsäure mehrmals mit Wasser gewaschen, 1,2-Dichlorpropan abdestilliert und anschließend in einer 40 cm Füllkörper-Kolonne, gefüllt mit 4 mm Glas Raschingringen, fraktioniert. Es wurden 18,5 g 2-(1,2-Dichloräthyl-)-oxiran mit einer Reinheit von 99,4 % isoliert.

Beispiel 2.

Herstellung von 2,3-Bis-(chlormethyl)-oxiran aus 1,4-Dichlor-2-buten und Perpropionsäure.

Zu 55,2 (0,4416 Mol) 1,4-Dichlor-2-buten wurden bei 70°C 63,97 g (0,147 Mol) Perpropionsäure als ca. 20 %ige Lösung in 1,2-Dichlorpropan zugetropft und anschließend bei dieser Temperatur weitergerührt. Nach 4 Stunden Reaktionszeit betrug der Persäure-Umsatz 94 %, nach 6 Stunden über 97 %. 2,3-Bis(chlormethyl)-oxiran wurde mit einer Selektivität, bezogen auf eingesetzte Perpropionsäure, von 94 % gebildet. Nach Entfernen der Propionsäure durch mehrmaliges Ausschütteln des reaktionsgemisches mit Wasser und anschließendes Abtrennen von 1,2-Dichlorpropan durch Destillation wurden nach der Fraktionierung über eine 30 cm Füllkörper-kolonne, gefüllt mit 4 mm Glasraschigringen, 19,0 g 2,3-Bis-(chlormethyl)-oxiran mit einer Reinheit von 99,9 % erhalten.

Beispiel 3.

Kontinuierliche Herstellung von 2,3-Bis-(chlormethyl)-oxiran aus 1,4-Dichlor-2-buten und Perpropionsäure.

Eine Lösung von Perpropionsäure in 1,2-Dichlorpropan, die mit einem Stabilisator vom Typ der im Handel erhältlichen Natriuimsalze von mit langkettigen Alkoholen partiell veresterten Polyphosphorsäuren versetzt ist, wurde mit 1,4-Dichlor-2-buten in einem als dreistufige Rührkesselkaskade ausgebildeten Reaktionssystem umgesetzt. Jeder der drei Rührkessel hatte ein Volumen von 9,4 l. Die Beheizung der Kessel erfolgte über im Kessel angebrachte Heizschlangen. Alle drei Kessel wurden auf 70°C thermostatisiert.

Diesem Reaktionssystem wurden pro Stunde 2.137,5 g Perpropionsäure als 20 %ige Lösung in 1,2-Dichlorpropan (4,75 Mol) und 1.781,25 g (14,25 Mol) 1,4-Dichlor-2-buten zugeführt, was einer mittleren Verweilzeit von etwa 8 Stunden entsprach. Unter diesen Reaktionsbedingungen wurde die Perpropionsäure zu 94,2 % umgesetzt. Die Selektivität des gebildeten 2,3-Bis-(chlormethyl)-oxirans betrug 92 %, bezogen auf eingesetzte perpropionsäure.

Das hinter dem dritten Reaktor anfallende Reaktionsgemisch besaß im Mittel folgende Zusammensetzung: 35,6 % 1,2-Dichlorpropan, 31,4 % 1,4-Dichlor-2-buten, 15,7 % 2,3-Bis-(chlormethyl)-oxiran und 17 % Propionsäure. Dieses Gemisch wurde zur Abtrennung der Propionsäure in einer pulsierenden Siebbodenkolonne mit der zweifachen Menge Wasser extrahiert. Danach betrug der Restgehalt an Propionsäure weniger als 0,1 %. Das nach dieser Operation erhaltene reaktionsgemisch wurde in einer Destillationsstraße aufgetrennt. In einer ersten Kolonne wurde 1,2-Dichlorpropan in einer Menge von 1.395 g pro Stunde abdestilliert. Das Sumpfprodukt dieser Kolonne, das im wesentlichen aus Ausgangsprodukt und Oxiran bestand, wurde in einer zweiten Kolonne unter vermindertem Druck aufgetrennt. Als Kopfprodukt wurden pro Stunde 1.235 g 1,4-Dichlor-2-buten erhalten. Das Sumpfprodukt dieser Kolonne wurde in einer dritten Kolonne unter vermindertem Druck von Hochsiedern befreit. Als Kopfprodukt wurden pro Stunde 606 g 2,3-Bis-(chlormethyl)-oxiran mit einer Reinheit von über 99,9 % erhalten. Dem entspricht eine Ausbeute von 90,5 %, bezogen auf die in das Reaktionssystem eingesetzte Perpropionsäure.

Beispiel 4.

Herstellung von 2,3-Bis-(brommethyl)-oxiran aus 1,4-Dibrom-2-buten und Perpropionsäure.

a) Bromierung von Butadien

171,4 g (3,17 Mol) Butadien wurden in 400 ml n-Hexan gelöst. Bei —20°C wurden unter Rühren 314 g (1,964 Mol) Brom zugetropft. Nach Zutropfende wurden noch 2 Stunden bei dieser Temperatur nachgerührt. Anschließend ließ man auf Raumtemperatur erwärmen und entfernte das Lösungsmittel im Vakuum. Es resultierten 380,4 g rohes Dibrombuten, wobei das Verhältnis, 1,4-Dibrom-2-buten zu 3,4-Dibrom-1-buten etwa 2 : 1 war. Die Isolierung des 1,4-Dibrom-2-butens erfolgte durch Destillation.

b) Umsetzung von 1,4-Dibrom-2-buten mit Perpropionsäure

Zu 64,2 g (0,3 Mol) 1,4-Dibrom-2-buten wurde bei 70°C unter Rühren 45 g 20 %ige Perpropionsäure in 1,2-Dichlorpropan (0,1 Mol) getropft und noch weitere 4 Stunden bei dieser Temperatur gerührt. Nach dieser Zeit betrug der Persäure-Umsatz 93 %. Die gaschromatographische Analyse ergab, daß das Epoxid mit einer Selektivität von 90,8 %, bezogen auf eingesetzte Perpropionsäure, gebildet wurde. Das Reaktionsgemisch wurde nach dem Abkühlen zur Entfernung der Propionsäure mehrmals mit Wasser gewaschen und fraktioniert. Es wurden 20,3 g Epoxid erhalten.

**Patentansprüche**

1. Verfahren zur Herstellung von halogenalkylsubstituierten Oxiranen aus halogenalkylsubstituierten Olefinen und Percarbonsäuren, dadurch gekennzeichnet, daß man ein chloralkyl- oder bromalkylsubstituierten Monoolefin der allgemeinen Formel

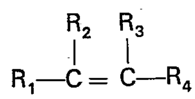

worin

$R_1$ und $R_4$ unabhängig voneinander Wasserstoff, $C_1$- bis $C_5$-Alkyl, $C_5$- bis $C_7$-Cycloalkyl, Monochlor-$C_1$- bis $C_5$-Alkyl, Monobrom-$C_1$- bis $C_5$-Alkyl, Dichlor-$C_1$- bis $C_5$-Alkyl, Dibrom-$C_1$- bis $C_5$-Alkyl, Monochlor-$C_5$- bis $C_7$-Cycloalkyl, Monobrom-$C_5$- bis $C_7$-Cycloalkyl, Dichlor-$C_5$- bis $C_7$-Cycloalkyl, Dibrom-$C_5$- bis $C_7$-Cycloalkyl bedeuten,

$R_2$ und $R_3$ unabhängig voneinander für Wasserstoff, $C_1$- bis $C_5$-Alkyl, Monochlor-$C_1$- bis $C_5$-Alkyl, Monobrom-$C_1$- bis $C_5$-Alkyl, Dichlor-$C_1$- bis $C_5$-Alkyl und Dibrom-$C_1$- bis $C_5$-Alkyl stehen, wobei die Reste

$R_2$ und $R_3$ zusammen mit den Kohlenstoffatomen der $C=C$ -Doppelbindung einen Ring mit bis zu 12 Kohlenstoffatomen bilden können,

wobei mindestens einer der Reste $R_1$ bis $R_4$ ein Dichlor oder Dibromrest der genannten Art ist oder wobei mindestens zwei der Reste $R_1$ bis $R_4$ ein Chlor oder Brom enthaltender Rest der genannten Art sind,

und wobei das Chloralkyl- oder Bromalkyl-substituierte Monoolefin mindestens 4 Kohlenstoffatome enthält,

mit einer Lösung einer 3 bis 4 Kohlenstoffatome enthaltenden Percarbonsäure in einem chlorierten, 1 bis 8 Kohlenstoffatome enthaltenden Kohlenwasserstoff bei einem Molverhältnis von Monoolefin zu Percarbonsäure wie 1,1 : 1 bis 10 : 1 und bei einer Temperatur von 30°C bis 100°C umsetzt, wobei die Percarbonsäure bis zu 5 Gew.-% Wasser und bis zu 2 Gew.-% Wasserstoffperoxid enthält und die zur Umsetzung gelangende Percarbonsäurelösung einen Mineralsäuregehalt unterhalb von 50 ppm besitzt.

2. Verfahren nach Anspruch 1, dadurch gekennzeichnet, daß man als chloralkyl- oder bromalkylsubstituiertes Monoolefin ein Olefin der Formel

$$R_5—CH=CH—R_6,$$

worin

$R_5$ und $R_6$ unabhängig voneinander $C_1$- bis $C_5$-Alkyl, Monochlor-$C_1$- bis $C_5$-Alkyl, Monobrom-$C_1$ bis $C_5$-

Alkyl, Dichlor-$C_1$- bis $C_5$- Alkyl oder Dibrom-$C_1$- bis $C_5$-Alkyl bedeuten, wobei die Reste $R_5$ und $R_6$ zusammen mit der Gruppe CH=CH zu einem Ring verknüpft sein können, und wobei mindestens einer der Reste $R_5$ und $R_6$ Dichlor-$C_1$- bis $C_5$-Alkyl oder Dibrom-$C_1$- bis $C_5$-Alkyl bedeutet oder beide Reste $R_5$ und $R_6$ Monochlor-$C_1$- bis $C_5$-Alkyl oder Monobrom-$C_1$- bis $C_5$-Alkyl bedeuten,
einsetzt.

3. Verfahren nach Anspruch 1 und 2, dadurch gekennzeichnet, daß man als chloralkyl- oder bromalkylsubstituiertes Monoolefin ein Olefin der Formel

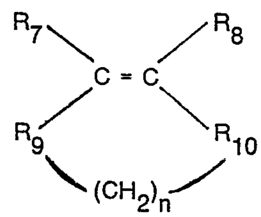

worin
$R_7$ und $R_8$ unabhängig voneinander Wasserstoff, $C_1$- bis $C_5$-Alkyl, Monochlor-$C_1$- bis $C_5$-Alkyl, Monobrom-$C_1$ bis $C_5$-Alkyl, Dichlor-$C_1$- bis $C_5$-Alkyl oder Dibrom-$C_1$- bis $C_5$-Alkyl bedeuten,
$R_9$ und $R_{10}$ unabhängig voneinander einen Methylen-, Chlormethylen-, Brommethylen-, 1,2-Dichloräthylen- oder 1,2-Dibrommethylen-Rest darstellen,
n für eine ganze Zahl von 1 bis 6 steht,
und wobei mindestens einer der Reste $R_7$ bis $R_{10}$ ein Dichlor- oder Dibromrest der genannten Art ist, oder wobei mindestens zwei der Reste $R_7$ bis $R_{10}$ ein Chlor oder Brom enthaltender Rest der genannten Art sind, einsetzt.

4. Verfahren nach Anspruch 1 und 2, dadurch gekennzeichnet, daß man als chloralkyl- oder bromalkylsubstituiertes Monoolefin ein Olefin der Formel

$$R_{11}—CH=CH—R_{12},$$

worin
$R_{11}$ und $R_{12}$ unabhängig voneinander Wasserstoff, $C_1$- bis $C_5$-Alkyl, Monochlor-$C_1$- bis $C_5$-Alkyl, Monobrom-$C_1$ bis $C_5$-Alkyl, Dichlor-$C_1$- bis $C_5$-Alkyl oder Dibrom-$C_1$- bis $C_5$-Alkyl bedeuten, wobei mindestens einer der Reste $R_{11}$ und $R_{12}$ für Dichlor-$C_1$- bis $C_5$-Alkyl oder Dibrom-$C_1$- bis $C_5$-Alkyl steht oder beide Reste $R_{11}$ und $R_{12}$ für Monochlor-$C_1$- bis $C_5$-Alkyl oder Monobrom-$C_1$- bis $C_5$-Alkyl stehen,
einsetzt.

5. Verfahren nach Anspruch 1, dadurch gekennzeichnet, daß man als chloralkyl- oder bromalkylsubstituiertes Monoolefin 1,4-Dichlor-2-buten einsetzt.

6. Verfahren nach Anspruch 1, dadurch gekennzeichnet, daß man als chloralkyl- oder bromalkylsubstituiertes Monoolefin 3,4-Dichlor-1-buten einsetzt.

7. Verfahren nach Anspruch 1, dadurch gekennzeichnet, daß man als chloralkyl- oder bromalkylsubstituiertes Monoolefin 1,4-Dibrom-2-buten einsetzt.

8. Verfahren nach Anspruch 1 bis 7, dadurch gekennzeichnet, daß man als Percarbonsäure Perpropionsäure einsetzt.

9. Verfahren nach Anspruch 1 bis 7, dadurch gekennzeichnet, daß man als Percarbonsäure Perisobuttersäure einsetzt.

10. Verfahren nach Anspruch 1 bis 9, dadurch gekennzeichnet, daß man als chlorierten Kohlenwasserstoff Dichlorpropan verwendet.

11. Verfahren nach Anspruch 1 bis 10, dadurch gekennzeichnet, daß man die Umsetzung bei einem Molverhältnis von Olefin zur Persäure wie 1,5 bis 3 : 1 durchführt.

12. Verfahren nach Anspruch 1 bis 11, dadurch gekennzeichnet, daß man die Umsetzung bei einer Temperatur von 60 bis 80°C durchführt.

13. Verfahren nach Anspruch 1 bis 12, dadurch gekennzeichnet, daß man das Reaktionsgemisch von der destillativen Aufarbeitung zur Abtrennung der bei der Umsetzung gebildeten der Percarbonsäure entsprechenden Carbonsäure, mit Wasser extrahiert.

**Claims**

1. Process for the preparation of halogenoalkyl-substituted oxiranes from halogenoalkyl-substituted olefines and percarboxylic acids, characterised in that a chloroalkyl- or bromoalkyl-substituted monoolefine of the general formula

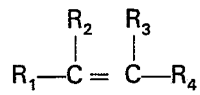

wherein
$R_1$ and $R_4$ independently of one another denote hydrogen, $C_1$- to $C_5$-alkyl, $C_5$- to $C_7$-cycloalkyl, monochloro-$C_1$- to $C_5$-alkyl, monobromo-$C_1$- to $C_5$-alkyl, dichloro-$C_1$- to $C_5$-alkyl, dibromo-$C_1$- to $C_5$-alkyl, monochloro-$C_5$- to $C_7$-cycloalkyl, monobromo-$C_5$- to $C_7$-cycloalkyl, dichloro-$C_5$- to $C_7$-cycloalkyl or dibromo-$C_5$- to $C_7$-cycloalkyl and
$R_2$ and $R_3$ independently of one another represent hydrogen, $C_1$- to $C_5$-alkyl, monochloro-$C_1$- to $C_5$-alkyl, monobromo-$C_1$- to $C_5$-alkyl, dichloro-$C_1$- to $C_5$-alkyl and dibromo-$C_1$- to $C_5$-alkyl,
or wherein
the radicals $R_2$ and $R_3$, together with the carbon atoms of the C=C double bond, can form a ring with up to 12 carbon atoms, at least one of the radicals $R_1$ to $R_4$ being a dichloro or dibromo radical of the type mentioned, or at least two of the radicals $R_1$ to $R_4$ being a chlorine- or bromine-containing radical of the type mentioned, and the chloroalkyl- or bromoalkyl-substituted monoolefine containing at least 4 carbon atoms,
is reacted with a solution of a percarboxylic acid containing 3 to 4 carbon atoms in a chlorinated hydrocarbon containing 1 to 8 carbon atoms at a molar ratio of monoolefine to percarboxylic acid of 1.1 : 1 to 10 : 1 and at a temperature of 30°C to 100°C, the percarboxylic acid containing up to 5% by weight of water and up to 2% by weight of hydrogen peroxide, and the percarboxylic acid solution used for the reaction having a mineral acid content of below 50 ppm.

2. Process according to Claim 1, characterised in that an olefine of the formula

$$R_5—CH=CH—R_6$$

wherein
$R_5$ and $R_6$ independently of one another denote $C_1$- to $C_5$-alkyl, monochloro-$C_1$- to $C_5$-alkyl, monobromo-$C_1$- to $C_5$-alkyl, dichloro-$C_1$ to $C_5$-alkyl or dibromo-$C_1$- to $C_5$-alkyl,
or wherein
the radicals $R_5$ and $R_6$, together with the group CH=CH, can be linked to form a ring, at least one of the radicals $R_5$ and $R_6$ denoting dichloro-$C_1$- to $C_5$-alkyl or dibromo-$C_1$- to $C_5$-alkyl or both radicals $R_5$ and $R_6$ denoting monochloro-$C_1$- to $C_5$-alkyl or monobromo-$C_1$ to $C_5$-alkyl,
is employed as the chloroalkyl- or bromoalkyl-substituted monoolefine.

3. Process according to Claim 1 and 2, characterised in that an olefine of the formula

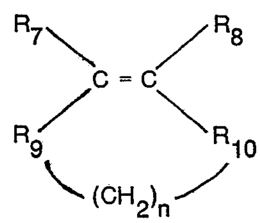

wherein
$R_7$ and $R_8$ independently of one another denote hydrogen, $C_1$- to $C_5$-alkyl, monochloro-$C_1$- to $C_5$-alkyl, monobromo-$C_1$ to $C_5$-alkyl, dichloro-$C_1$- to $C_5$-alkyl or dibromo-$C_1$- to $C_5$-alkyl,
$R_9$ and $R_{10}$ independently of one another represent a methylene, chloromethylene, bromomethylene, 1,2-dichloroethylene, or 1,2-dibromomethylene radical and
n represents an integer from 1 to 6, at least one of the radicals $R_7$-$R_{10}$ being a dichloro or dibromo radical of the type mentioned or at least two of the radicals $R_7$ to $R_{10}$ being a chlorine- or bromine-containing radical of the type mentioned,
is employed as the chloroalkyl or bromoalkyl-substituted monoolefine.

4. Process according to Claim 1 and 2, characterised in that an olefine of the formula

$$R_{11}8CH=CH—R_{12}$$

wherein
$R_{11}$ and $R_{12}$ independently of one another denote hydrogen $C_1$- to $C_5$-alkyl, monochloro-$C_1$- to $C_5$-alkyl, monobromo-$C_1$- to $C_5$-alkyl, dichloro-$C_1$-to $C_5$-alkyl or dibromo-$C_1$- to $C_5$-alkyl, at least one of the radicals $R_{11}$ and $R_{12}$ representing dichloro-$C_1$- to $C_5$-alkyl or dibromo-$C_1$- to $C_5$-alkyl or both radicals $R_{11}$ and $R_{12}$ representing monochloro-$C_1$- to $C_5$-alkyl or monobromo-$C_1$- to $C_5$-alkyl,
is employed as the chloroalkyl- or bromoalkyl-substituted monoolefine.

5. Process according to Claim 1, characterised in that 1,4-dichloro-2-butene is employed as the chloroalkyl- or bromoalkyl-substituted monoolefine.

6. Process according to Claim 1, characterised in that 3,4-dichloro-1-butene is employed as the chloroalkyl- or bromoalkyl-substituted monoolefine.

7. Process according to Claim 1, characterised in that 1,4-dibromo-2-butene is employed as the chloroalkyl- or bromoalkyl-substituted monoolefine.

8. Process according to Claim 1 to 7, characterised in that perpropionic acid is employed as the percarboxylic acid.

9. Process according to Claim 1 to 7, characterised in that perisobutyric acid is employed as the percarboxylic acid.

10. Process according to Claim 1 to 9, characterised in that dichloropropane is used as the chlorinated hydrocarbon.

11. Process according to Claim 1 to 10, characterised in that the reaction mixture is carried out at a molar ratio of olefine to peracid 1.5 to 3 : 1.

12. Process according to Claim 1 to 11, characterised in that the reaction is carried out at a temperature of 60 to 80°C.

13. Process according to Claim 1 to 12, characterised in that the reaction mixture is extracted with water before distillative working up in order to separate off the carboxylic acid which is formed during the reaction and corresponds to the percarboxylic acid.

**Revendications**

1. Procédé de production d'oxiranes à substituants halogénalkyl à partir d'oléfines à substituants halogénalkyle et d'acides percarboxyliques, caractérisé en ce qu'on fait réagir une mono-oléfine à substituant chloralkyle ou bromalkyle de formule générale:

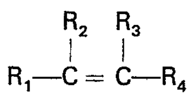

dans laquelle:

$R_1$ et $R_4$ représentent, indépendamment l'un de l'autre, de l'hydrogène, un radical alkyle en $C_1$ à $C_5$, cycloalkyle en $C_5$ à $C_7$, monochloralkyle en $C_1$ à $C_5$, monobromalkyle en $C_1$ à $C_5$, dichloralkyle en $C_1$ à $C_5$, dibromalkyle en $C_1$ à $C_5$, monochlorocycloalkyle en $C_5$ à $C_7$, monobromocycloalkyle en $C_5$ en $C_7$, dichlorocycloalkyle en $C_5$ à $C_7$, dibromocycloalkyle en $C_5$ à $C_7$,

$R_2$ et $R_3$ représentent, indépendamment l'un de l'autre, de l'hydrogène, un radical alkyle en $C_1$ à $C_5$, monochloralkyle en $C_1$ à $C_5$, monobromoalkyle en $C_1$ à $C_5$, dichloralkyle en $C_1$ à $C_5$, dibromalkyle en $C_1$ à $C_5$, les restes

$R_2$ et $R_3$ pouvant former conjointement avec les atomes de carbone de la double liaison C=C un noyau ayant jusqu'à 12 atomes de carbone,

au moins l'un des restes $R_1$ à $R_4$ étant un reste dichloro ou dibromo du type mentionné ou bien au moins deux des restes $R_1$ à $R_4$ étant un reste contenant du chlore ou du brome du type mentionné,

et la mono-oléfine à substituant chloralkyle ou bromalkyle contenant au moins 4 atomes de carbone, avec une solution d'un acide percarboxylique contenant 3 ou 4 atomes de carbone dans un hydrocarbure chloré contenant 1 à 8 atomes de carbone dans un rapport molaire de la mono-oléfine à l'acide percarboxylique de 1,1 : 1 à 10 : 1 et à une température de 30 à 100°C, l'acide percarboxylique contenant jusqu'à 5 % en poids d'eau et jusqu'à 2 % en poids de peroxyde d'hydrogène et la solution d'acide percarboxylique mise en réaction ayant une teneur en acide minéral inférieure à 50 ppm.

2. Procédé suivant la revendication 1, caractérisé en ce qu'on utilise comme mono-oléfine à substituant chloralkyle ou bromalkyle une oléfine de formule:

$$R_5—CH=CH—R_6$$

dans laquelle:

$R_5$ et $R_6$ représentent, indépendamment l'un de l'autre, un radical alkyle en $C_1$ à $C_5$, monochloralkyle en $C_1$ à $C_5$, monobromalkyle en $C_1$ à $C_5$, dichloralkyl en $C_1$ à $C_5$ ou dibromalkyle en $C_1$ à $C_5$,

les restes $R_5$ et $R_6$ pouvant être liés en un noyau conjointement avec le groupe CH=CH, et au moins l'un des restes $R_5$ et $R_6$ représentant un radical dichloralkyle en $C_1$ à $C_5$ ou dibromalkyle en $C_1$ à $C_5$ ou bien les deux restes $R_5$ et $R_6$ représentent un reste monochloralkyle en $C_1$ à $C_5$ ou monobromalkyle en $C_1$ à $C_5$.

3. Procédé suivant les revendications 1 et 2, caractérisé en ce qu'on utilise comme mono-oléfine un substituant chloralkyle ou bromalkyle, une oléfine de formule:

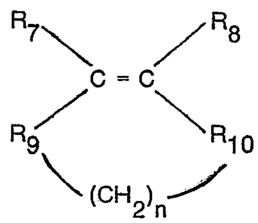

dans laquelle:

$R_7$ et $R_8$ représentent, indépendamment l'un de l'autre, de l'hydrogène, un radical alkyle en $C_1$ à $C_5$, monochloralkyle en $C_1$ à $C_5$, monobromalkyle en $C_1$ à $C_5$, dichloralkyle en $C_1$ à $C_5$ ou dibromalkyle en $C_1$ à $C_5$,

$R_9$ et $R_{10}$ représentent, indépendamment l'un de l'autre, un reste méthylène, chlorométhylène, bromométhylène, 1,2-dichloréthylène ou 1,2-dibromométhylène,

*n* est un nombre entier de 1 à 6,

et au moins l'un des restes $R_7$ à $R_{10}$ représentent un reste dichloro ou dibromo du type mentionné ou bien au moins deux des restes $R_7$ à $R_{10}$ représentant un reste du type mentionné contenant du chlore ou du brome.

4. Procédé suivant les revendications 1 et 2, caractérisé en ce qu'on utilise comme mono-oléfine à substituant chloralkyle ou bromalkyle, une oléfine de formule:

$$R_{11}—CH=CH—R_{12}$$

dans laquelle:

$R_{11}$ et $R_{12}$ représentent, indépendamment l'un de l'autre, de l'hydrogène, un reste alkyle en $C_1$ à $C_5$, monochloralkyle en $C_1$ à $C_5$, monobromalkyle en $C_1$ à $C_5$, dichloralkyle en $C_1$ à $C_5$ ou dibromalkyle en $C_1$ à $C_5$,

l'un au moins des restes $R_{11}$ et $R_{12}$ étant un reste dichloralkyle en $C_1$ à $C_5$ ou dibromalkyle en $C_1$ à $C_5$ ou bien les deux restes $R_{11}$ et $R_{12}$ représentant un reste monochloralkyle en $C_1$ à $C_5$ ou monobromalkyle en $C_1$ à $C_5$.

5. Procédé suivant la revendication 1, caractérisé en ce qu'on utilise le 1,4-dichloro-2-butène comme mono-oléfine à substituant chloralkyle ou bromalkyle.

6. Procédé suivant la revendication 1, caractérisé en ce qu'on utilise le 3,4-dichloro-1-butène comme mono-oléfine à substituant chloralkyle ou bromalkyle.

7. Procédé suivant la revendication 1, caractérisé en ce qu'on utilise le 1,4-dibromo-2-butène comme mono-oléfine à substituant chloralkyle ou bromalkyle.

8. Procédé suivant les revendications 1 à 7, caractérisé en ce qu'on utilise l'acide perpropionique comme acide percarboxylique.

9. Procédé suivant les revendications 1 à 7, caractérisé en ce qu'on utilise l'acide perisobutyrique comme acide percarboxylique.

10. Procédé suivant les revendications 1 à 9, caractérisé en ce qu'on utilise le dichloropropane comme hydrocarbure chloré.

11. Procédé suivant les revendications 1 à 10, caractérisé en ce qu'on conduit la réaction à un rapport molaire de l'oléfine au peracide de 1,5 à 3 : 1.

12. Procédé suivant les revendications 1 à 11, caractérisé en ce qu'on conduit la réaction à une température de 60 à 80°C.

13. Procédé suivant les revendications 1 à 12, caractérisé en ce qu'on extrait le mélange réactionnel à l'eau avant le traitement de distillation pour séparer l'acide carboxylique formé lors de la réaction, correspondant à l'acide percarboxylique.